# FASCICULE DE BREVET EUROPEEN

(11) **EP 0 828 528 B1**
(45) Date de publication et mention de la délivrance du brevet: **04.08.1999**
(21) Numéro de dépôt: 96917553.8
(22) Date de dépôt: 24.05.1996
(51) Int. Cl.: A61M 5/32, A61M 5/50

(54) **SERINGUE PRE-REMPLIE DE SECURITE**
VORGEFÜLLTE SICHERHEITSSPRITZE
PREFILLED SAFETY SYRINGE

(30) Priorité: 24.05.1995 FR 9506415
(43) Date de publication de la demande: 18.03.1998
(73) Titulaire: Biodome, 63500 Issoire (FR)
(72) Inventeur: ANEAS, Antoine, F-63500 Ménétrol (FR)
(74) Mandataire: Myon, Gérard Jean-Pierre
(86) Numéro de dépôt international: FR9600789
(87) Numéro de publication internationale: WO9637247

(56) Documents cités:
- WO-A-92/15351
- WO-A-93/02728
- US-A- 4 747 837
- US-A- 5 085 647
- US-A- 5 267 972
- US-A- 5 366 447

## Description

La présente invention concerne un dispositif à usage médical, du type "ampoule - seringue", c'est-à-dire comprenant ou constitué par une seringue pré-remplie et prête à l'emploi, et à usage unique.

Conformément à la demande de brevet internationale, publiée sous le numéro WO-A-93/02728, on a décrit, un tel dispositif, qui comprend :
- un corps tubulaire contenant le liquide médicamenteux à injecter, transparent ou non ;
- un nez de réception d'une aiguille creuse communiquant avec l'intérieur du corps tubulaire ; et
- un piston coulissant, à l'opposé dudit nez, à l'intérieur du corps tubulaire, actionnable à son extrémité extérieure par son utilisateur, disposé par rapport au corps tubulaire dans une position prédéterminée, dans laquelle son extrémité intérieure détermine avec le reste dudit corps un volume utile interne, au moins partiellement rempli avec une charge du liquide médicamenteux ;
- une coiffe de protection après injection, coaxiale avec et montée de manière coulissante sur le corps, dont la longueur est suffisante pour recouvrir complètement l'aiguille, dans une position avancée, saillant à l'avant du corps, disposée dans une position escamotée sur ledit corps, un capuchon de protection du nez du corps au moins, et des moyens d'inviolabilité, notamment une ligne annulaire de déchirure à faible résistance.

Le problème principal du dispositif précédemment décrit est qu'il est toujours possible de réutiliser la seringue, en réinsérant une nouvelle cartouche ou carpule, ce qui nuit à sa sécurité et limite son champ d'application.

Conformément au brevet américain publié sous le numéro US-A-4 747 837, un dispositif similaire est décrit, avec la différence que la seringue n'est pas préremplie et le capuchon est monté à l'intérieur de la coiffe sur le nez du corps par frottement, et non par des moyens d'inviolabilité. Le dispositif comporte également des moyens d'arrêt de la coiffe dans sa position avancée empéchant toute utilisation ultérieure. Toutefois, il est possible de déplacer la coiffe vers l'avant et de la bloquer par mégard, rendant ainsi le dispositif inutilisable.

La présente invention a pour objet un dispositif tel que précédemment défini, qui intégre des moyens d'inviolabilité assurant à la fois l'étanchéité de l'embout de la seringue contre toute contamination, et la protection de l'aiguille d'injection contre toute piqûre accidentelle, une fois le dispositif utilisé.

La présente invention a également pour but de résoudre les problèmes des dispositifs de l'art antérieur, en prévoyant un dispositif à usage médical, tel qu'une seringue, jetable, et à usage unique, et permettant de s'apercevoir que l'étanchéité du dispositif a été violée. Par ailleurs, la présente invention a pour but de fournir un dispositif tel que décrit précédemment qui oblige l'utilisateur de suivre un certain fonctionnement, évitant toute fausse manipulation, et fixant par sa construction son ergonomie.

Conformément à la présente invention, le capuchon comprend en outre à sa base un jonc annulaire engagé de manière fixe sur le corps tubulaire du côté de son nez et relié au reste dudit capuchon par les moyens d'inviolabilité, et le capuchon est agencé ou disposé par rapport à la coiffe, en sorte que le premier bloque tout mouvement coulissant de la seconde, restant dans sa position escamotée, tant que ledit capuchon n'est pas séparé du corps tubulaire. Pour ce faire, le capuchon peut, par exemple, être lié à la coiffe par les moyens d'inviolabilité. Ce jonc peut également être agencé ou disposé par rapport à la coiffe, pour arrêter le mouvement coulissant de cette dernière, dans sa position avancée.

L'avantage du dispositif de la présente invention est que grâce à sa construction particulière, il devient impossible de changer la séquence des étapes d'utilisation, et la seringue ne peut pas être réutilisée.

En outre, on peut prévoir des moyens d'arrêt de la coiffe dans sa position escamotée et/ou avancée, entre le corps tubulaire et ladite coiffe.

Dans un mode d'exécution préféré de l'invention, le dispositif comprend une aiguille creuse d'injection disposée dans le nez du corps tubulaire. Dans ce cas le capuchon peut, par exemple, avoir une longueur suffisante pour protéger complètement ladite aiguille, avant injection, et comporte un bouchon dans lequel l'extrémité d'injection de l'aiguille est fichée, disposé à l'intérieur du capuchon à l'opposé du corps tubulaire.

La présente invention est maintenant décrite par référence aux dessin annexé, dans lequel :
- la Figure 1 représente une vue en coupe d'un dispositif selon l'invention, prêt à l'emploi ;
- la Figure 2 représente, toujours en coupe, le dispositif de la figure 1, prêt pour une injection par piqûre sur un patient ;
- la Figure 3 représente le dispositif des figures 1 et 2, toujours en coupe, après injection sur le patient ;
- la Figure 4 représente à l'échelle agrandie, les positions relatives de l'extrémité distale de la coiffe et l'extrémité proximale du capuchon ;
- la Figure 5 montre une variante d'un dispositif selon l'invention, dans laquelle le capuchon est monté sur le nez du corps, et non sur le corps lui-même ;
- la Figure 6 montre le dispositif de la Figure 5, après enlèvement du capuchon ;
- la Figure 7 montre le dispositif des Figures 5 et 6, après coulissement et bloquage de la coiffe dans sa position avancée, pour empêcher toute réutilisation du dispositif ;
- la Figure 8 montre, à l'échelle agrandie, une partie du dispositif de la Figure 5.

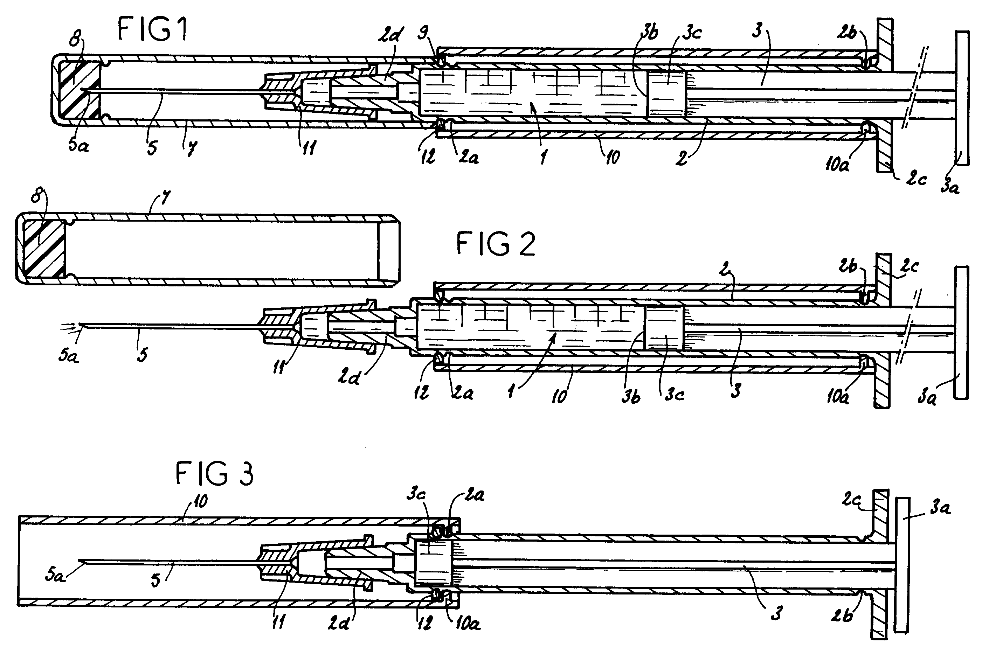

Un dispositif selon la présente invention est réalisé et obtenu par moulage des différentes pièces ou composants le constituant, à l'exception bien entendu de l'aiguille creuse d'injection. Le dispositif (1) conformément aux figures 1 à 3 comprend de manière générale :
- un corps tubulaire 2 en matière plastique transparente ou translucide, pourvu éventuellement d'une graduation, contenant un liquide médicamenteux à injecter ; ce corps est pourvu à une extrémité de deux oreilles perpendiculaires de préhension 2c, et à l'autre extrémité d'un nez 2d, servant à l'assemblage avec l'aiguille creuse d'injection décrite ci-après ; deux rainures annulaires 2a et 2b sont ménagées à l'extérieur du corps 2, respectivement du côté des oreilles 2c et du côté du nez 2d, pour coopérer avec la coiffe décrite ci-après ;
- un piston 3 coulissant à l'intérieur du corps 2, actionnable à son extrémité extérieure 3a par l'utilisateur, et comportant un noyau 3c formant piston ; ce piston est disposé par rapport au corps tubulaire 2, dans une position dans laquelle son extrémité intérieure 3b détermine un volume utile interne, rempli avec une charge du liquide médicamenteux ;
- une aiguille creuse 5 d'injection, disposée à l'extrémité du corps tubulaire 2 opposée au piston, montée coaxialement par rapport au corps précité grâce à un raccord 11 ; cette aiguille 5 communique donc avec l'intérieur du corps 2 ;
- un capuchon 7 de protection de l'aiguille 5 avant injection, ainsi que d'obturation de son extrémité ouverte 5a d'injection, enfermant donc l'aiguille 5, mais monté de manière séparable sur le corps tubulaire 2. Ce capuchon 7 comporte un bouchon 8, par exemple en caoutchouc, dans lequel l'extrémité 5a d'injection de l'aiguille 5 est fichée, le bouchon 8 étant disposé à l'intérieur du capuchon 7, à l'opposé du corps tubulaire 2 ;
- une coiffe 10 de protection après injection est montée de manière coulissante sur, et coaxiale avec le corps tubulaire 2, dont la longueur est suffisante pour recouvrir l'aiguille 5 d'injection, dans la position avancée saillante définie ci-après ; cette coiffe est disposée dans une position escamotée sur le corps tubulaire, et est susceptible d'être déplacée dans une position saillant à l'avant du corps précité, pour protéger l'aiguille 5 d'injection, après la séparation complète du capuchon, et l'utilisation de la seringue.

Le capuchon comprend à sa base un jonc 12 annulaire, engagé de manière fixe en rotation sur le corps tubulaire 2, du côté de son nez 2d. Ce jonc est relié au capuchon 7 par des moyens d'inviolabilité séparables sous l'action de l'utilisateur, par exemple par des ponts de matière plastique, définissant une ligne 9 annulaire de déchirure à faible résistance. De manière non représentée, le jonc 12 et le corps 2 sont engagés l'un sur l'autre grâce à un crantage annulaire prévu sur ledit corps et des crans correspondants disposés à l'intérieur du jonc 12, de telle sorte qu'il est possible de séparer le capuchon 7 du jonc 12, par simple rotation de l'un par rapport à l'autre.

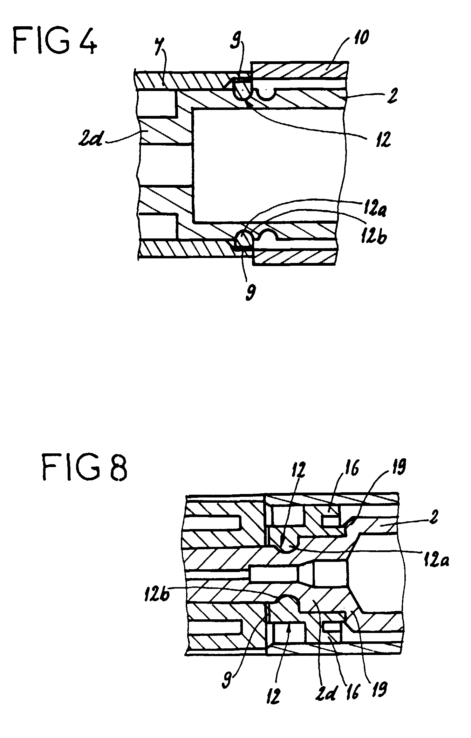

Comme illustré à la Figure 4, à l'échelle agrandie, le capuchon 7 est agencé par rapport à la coiffe 10, en sorte que le premier bloque tout mouvement de la seconde, restant dans sa position escamotée, tant que le capuchon 7 n'a pas été séparé du corps tubulaire 2. Et le capuchon 7 est agencé par rapport à la coiffe 10 pour arrêter le déplacement de cette dernière, dans sa position avancée, saillant à l'avant du corps tubulaire 2.

Grâce à une nervure 10a, homologue des rainures 2a et 2b du corps tubulaire 2, il est possible de maintenir la coiffe 10 dans sa position escamotée, grâce à l'encliquetage de la nervure 10a avec la rainure 2b, et dans sa position avancée saillante grâce à l'encliquetage de la nervure 10a avec une rainure 2a.

L'utilisation du dispositif décrit précédemment est la suivante :
- au départ, selon la figure 1, le dispositif est prêt à l'emploi : la coiffe 10 est escamotée, et le capuchon 7 est solidarisé sur le corps 2, en obturant l'extrémité 5a de l'aiguille 5 avec le bouchon 8, et protégeant complètement cette dernière.

Par rotation du capuchon 7 par rapport au jonc 12, on sépare ledit capuchon du corps 2, en laissant apparaître l'aiguille d'injection 5, ce qui permet de pratiquer l'injection sur le patient, en poussant le piston 3.

Après injection, on déplace la coiffe 10 dans sa position avancée saillante, qui devient définitive, en sorte que l'aiguille 5 est totalement comprise dans la coiffe, et ainsi protégée.

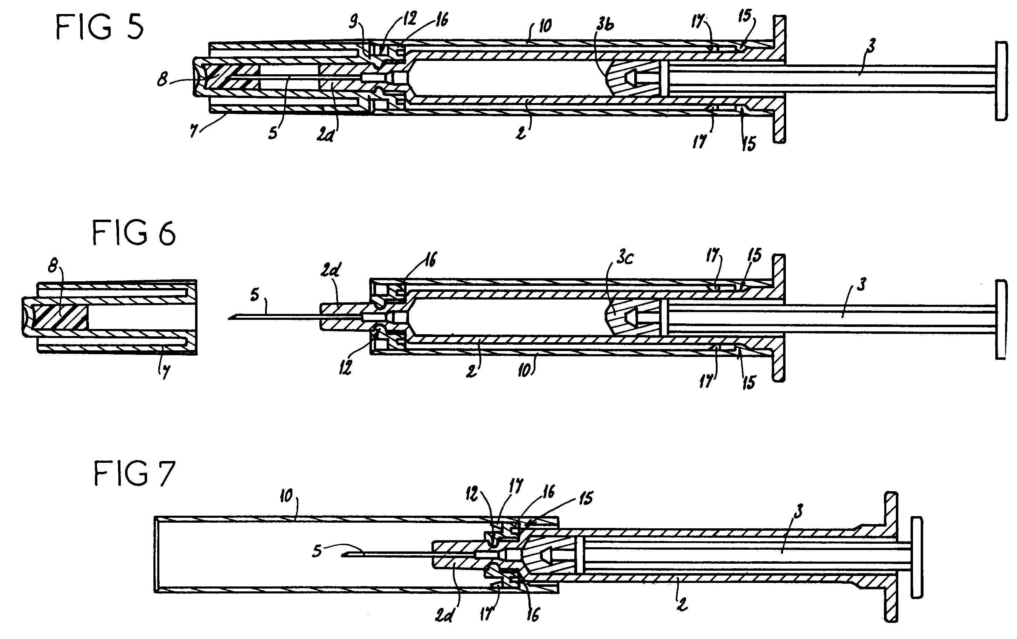

Les variantes de l'invention telles qu'illustrées aux Figures 5 à 8 seront maintenant décrites uniquement par rapport aux différences que celles-ci présentent avec le mode d'exécution des Figures 1 à 4. Les numéros de référence utilisés resteront les mêmes pour les éléments communs à tous les modes d'exécution.

Le dispositif représenté par les figures 5 à 8 ne diffère qu'en ce que l'aiguille 5 n'est pas montée sur un raccord mais est fixée directement dans le nez 2d, par exemple, par collage ou soudure, et communique ainsi directement avec le volume interne du corps tubulaire 2. Dans ce cas, le capuchon 7 a une longueur suffisante pour protéger complètement l'aiguille avant injection et est muni d'un bouchon 8, dans lequel l'extrémité 5a de l'aiguille 5 est fichée, disposé à l'intérieur du capuchon 7 à l'opposé du corps tubulaire. Le capuchon 7 comprend également un jonc 12 qui est solidaire de ce dernier, et qui se sépare de celui-ci, lorsqu'une force de déchirure est appliquée sur la ligne annulaire de faible résistance 9. Comme cela est mieux illlustré en Figure 8, le jonc 12 est fixé sur le nez 2d du corps tubulaire 2 par le biais d'un bourrelet annulaire 12a qui s'encliquete dans une rainure annulaire 12b correspondante prévue sur le nez 2d. Le jonc 12 comporte en outre une saillie ou bride annulaire 16 s'étendant entre le corps 2 et la coiffe 10 en direction de l'extrémité du piston, jusqu'à l'épaule 19 du corps 2. La coiffe 10 comporte une saillie annulaire 17 chanfreinée vers l'avant, de sorte qu'en avançant, la saillie 17 de la coiffe dépasse la bride annulaire 16 du jonc 12 et se loge à l'avant de cette dernière, tout mouvement en translation ultérieur étant bloqué par le bourrelet annulaire constituant pour partie les moyens d'arrêt 15 de la coiffe dans sa position escamotée, comme cela sera décrit ci-après. Ces moyens d'arrêt 15 de la coiffe 10 dans sa position escamotée, prévus entre le corps tubulaire 2 et la coiffe 10, sont constitués par exemple par un bourrelet annulaire moulé dans la coiffe 10, chanfreiné du côté de l'extrémité du piston, venant en prise élastique avec le corps 2 et empêchant tout mouvement de cette dernière. Après injection, la coiffe 10 est avancée et arrêtée dans sa position saillante recouvrant complètement l'aiguille 5 par la bride annulaire 16 servant de moyen d'arrêt de la coiffe 10 dans sa position avancée et le chanfrein du bourrelet annulaire des moyens d'arrêt 15 qui s'encliquète dans l'espace d'arrêt entre la bride 10 et l'épaule 19 du corps 2.

## Revendications

1. Dispositif à usage médical, du type seringue (1) pré-remplie prête à l'emploi, comprenant un corps tubulaire (2) avec un nez (2d) de réception d'une aiguille creuse (5) d'injection communiquant avec l'intérieur dudit corps, un piston (3) coulissant à l'opposé dudit nez, à l'intérieur du corps (2), actionnable à son extrémité extérieure (3a) par l'utilisateur, disposé par rapport au corps (2) dans une position (Fig. 1, 2) dans laquelle son extrémité intérieure (3b) détermine un volume utile interne, au moins partiellement rempli par une charge d'un liquide médicamenteux, une coiffe (10) de protection après injection, coaxiale avec et montée de manière coulissante sur le corps (2), dont la longueur est suffisante pour recouvrir complètement l'aiguille (5), dans une position avancée, saillant à l'avant du corps (2), disposée dans une position escamotée sur ledit corps (2) et un capuchon (7) de protection du nez (2d) du corps (2) au moins, et des moyens d'inviolabilité, notamment une ligne (9) annulaire de déchirure à faible résistance, **caractérisé en ce que** le capuchon (7) comprend en outre à sa base un jonc annulaire (12) engagé de manière fixe sur le corps tubulaire (2) du côté de son nez (2d) et relié au reste dudit capuchon par les moyens d'inviolabilité, et le capuchon (7) est agencé ou disposé par rapport à la coiffe (10), en sorte que le premier bloque tout mouvement coulissant de la seconde, restant dans sa position escamotée, tant que ledit capuchon n'est pas séparé du corps tubulaire (2).

2. Dispositif selon la revendication 1, caractérisé en ce que le jonc (12) du capuchon est agencé ou disposé par rapport à la coiffe (10), pour arrêter le mouvement coulissant de cette dernière, dans sa position avancée.

3. Dispositif selon la revendication 1, caractérisé en ce que des moyens (2b, 10a) d'arrêt de la coiffe (10) dans sa position escamotée, sont prévus entre le corps tubulaire (2) et ladite coiffe (10).

4. Dispositif selon la revendication 1, caractérisé en ce que des moyens (2a, 10a) d'arrêt de la coiffe (10) dans sa position avancée, sont prévus entre le corps tubulaire (2) et ladite coiffe.

5. Dispositif selon la revendication 1, comprenant l'aiguille creuse (5) d'injection disposée dans le nez (2d) du corps tubulaire, caractérisé en ce que le capuchon (7) a une longueur suffisante pour protéger complètement ladite aiguille, avant injection, et comporte un bouchon (8) dans lequel l'extrémité (5a) d'injection de l'aiguille (5) est fichée, disposé à l'intérieur du capuchon (7) à l'opposé du corps tubulaire (2).

## Claims

1. Device for medical use, of the ready-to-use prefilled syringe (1) type, comprising:-a tubular body (2) with a nose (2d) for receiving a hollow injection needle (5) communicating with the inside of the said body; a sliding plunger (3) which lies at the opposite end from the said nose, inside the body (2), and can be activated at its outer end (3a) by the user and is arranged, in relation to the body (2), in a position (Figs. 1, 2) in which its inner end (3b) defines an internal useful volume at least partially filled with a charge of a liquid medication; a cover (10) for protection after injection, which is coaxial with the body (2) and mounted so as to slide thereon and whose length is sufficient to completely cover the needle (5) in an advanced position protruding forwards of the body (2), arranged in a retracted position on the said body (2); and a cap (7) for protecting the nose (2d) of the body (2) at least; and tamperproofing means, in particular an annular tear line (9) of low strength; characterized in that the cap (7) additionally comprises at its base an annular retaining ring (12) engaged in a fixed manner on the tubular body (2) at the end towards its nose (2d) and connected to the rest of the said cap via the tamperproofing means; and the cap (7) is arranged or disposed in relation to the cover (10) in such a way that the former blocks any sliding movement of the latter, remaining in its retracted position, as long as the said cap is not separated from the tubular body (2).

2. Device according to Claim 1, characterized in that the retaining ring (12) of the cap is arranged or disposed in relation to the cover (10) so as to stop the sliding movement of the latter in its advanced position.

3. Device according to Claim 1, characterized in that means (2b, 10a) for stopping the cover (10) in its retracted position are provided between the tubular body (2) and the said cover (10).

4. Device according to Claim 1, characterized in that means (2a, 10a) for stopping the cover (10) in its advanced position are provided between the tubular body (2) and the said cover.

5. Device according to Claim 1, comprising the hollow injection needle (5) disposed in the nose (2d) of the tubular body, characterized in that the cap (7) has a sufficient length to protect the said needle completely, before injection, and includes a stopper (8) in which the injection end (5a) of the needle (5) is engaged, this stopper being disposed inside the cap (7) opposite the tubular body (2).

## Patentansprüche

1. Vorrichtung zur medizinischen Benutzung vom Typ einer gebrauchsfertig vorgefüllten Spritze (1), enthaltend einen zylindrischen Körper oder Tubus (2), mit einer Spitze (2d) zur Aufnahme einer hohlen Injektionsnadel, die in Verbindung mit dem Inneren des genannten Körper steht, einen innerhalb des Körpers (2) angeordneten, verschiebbaren Kolben (3) an dem der Spitze gegenüberliegenden Ende, den der Benutzer am äußeren Ende (3a) angreifend bewegen kann und der bezüglich des Körpers (2) in eine Stellung (Fig. 1, 2) gebracht ist, in der sein inneres Ende (3b) ein inneres nutzbares Volumen begrenzt, das mindestens teilweise mit einer gewissen Menge eines flüssigen Medikaments oder Wirkstoffs gefüllt ist, eine Hülse (10) zum Schutz nach der Injektion, die koaxial zum und gleitfähig über den Körper (2) ausgebildet ist, deren Länge ausreichend ist, um die Nadel (5) völlig zu bemänteln oder zu umhüllen, wenn sie in einer vorgeschobenen, über den Körper (2) hinausragenden Stellung liegt, in einer zurückgezogenen Stellung über dem Körper (2), wenigstens für die Spitze (2d) des Körpers (2) eine Schutzkappe (7) und Mittel zum Sichern der Unversehrtheit, insbesondere eine ringförmige Abreißlinie (9) geringen Widerstands, dadurch gekennzeichnet, daß die Schutzkappe (7) außerdem an ihrer Basis einen Ring (12) enthält, der in der Nähe der Spitze (2d) fest an dem Körper (2) befestigt und mit dem Rest der Schutzkappe (7) über die Mittel zur Sicherung der Unversehrtheit verbunden ist, und daß die Schutzkappe (7) so bezüglich der Hülse (10) angeordnet ist, daß erstere jede Schubbewegung der zweiten verhindert, und diese in ihrer zurückgezogenen Stellung verbleibt, solange die Kappe nicht vom Körper (2) getrennt ist.

2. Vorrichtung nach Anspruch 1, dadurch gekennzeichnet, daß der Ring (12) der Schutzkappe bezüglich der Hülse (10) so ausgebildet ist, daß er eine Schiebebewegung letzterer in ihrer vorgerückten Stellung verhindert.

3. Vorrichtung nach Anspruch 1, dadurch gekennzeichnet, daß zwischen dem Körper (2) und der Hülse (10) Mittel (2b, 10a) zum Arretieren dieser Hülse (10) in ihrer eingezogenen Stellung vorgesehen sind.

4. Vorrichtung nach Anspruch 1, dadurch gekennzeichnet, daß zwischen dem Körper (2) und der Hülse (10) Mittel (2a, 10a) zum Arretieren dieser Hülse (10) in ihrer vorgeschobenen Stellung vorgesehen sind.

5. Vorrichtung nach Anspruch 1, enthaltend eine an der Spitze (2d) eines Tubes (2) angeordnete, hohle Injektionsnadel (5), dadurch gekennzeichnet, daß die Schutzkappe (7) eine ausreichende Länge hat, um vor der Injektion die genannte Nadel vollständig zu schützen, und daß sie einen im Inneren der Schutzkappe (7) gegenüber dem Tubus (2) angeordneten Zapfen (8) enthält, in den die Spitze (5a) der Injektionsnadel (5) gesteckt werden kann.
